# EUROPÄISCHE PATENTSCHRIFT

(11) **EP 0 918 515 B1**
(45) Veröffentlichungstag und Bekanntmachung des Hinweises auf die Patenterteilung: **27.04.2005**
(21) Anmeldenummer: 97923937.3
(22) Anmeldetag: 20.05.1997
(51) Int. Cl.: A61K 31/155, A61K 31/455, A61K 31/22, A61K 45/00, A61K 45/06, A61P 3/06

(54) **VERWENDUNG VON INHIBITOREN DES ZELLULÄREN Na+ /H+ -EXCHANGERS (NHE) ZUR HERSTELLUNG EINES MEDIKAMENTS ZUR NORMALISIERUNG DER SERUMLIPIDE**
USE OF INHIBITORS OF THE CELLULAR Na+ /H+ EXCHANGER (NHE) FOR PREPARING A MEDICAMENT FOR NORMALIZING SERUM LIPIDS
UTILISATION D'INHIBITEURS DE L'ECHANGEUR CELLULAIRE Na+ /H+ (NHE) POUR PREPARER UN MEDICAMENT PERMETTANT DE NORMALISER LES LIPIDES SERIQUES

(30) Priorität: 03.06.1996 DE 19622222; 26.03.1997 DE 19712636
(43) Veröffentlichungstag der Anmeldung: 02.06.1999
(73) Patentinhaber: Aventis Pharma Deutschland GmbH, 65929 Frankfurt am Main (DE)
(72) Erfinder: LANG, Hans, Jochen, D-65719 Hofheim (DE); JANSEN, Hans-Willi, D-65527 Niedernhausen (DE); SCHWARK, Jan-Robert, D-65929 Frankfurt (DE); KLEEMANN, Heinz-Werner, D-65474 Bischofsheim (DE); JUNG, Oliver, D-35633 Lahnau (DE); SCHÄFER, Hans-Ludwig, D-55252 Mainz (DE); LINZ, Wolfgang, D-55129 Mainz (DE); KRAMER, Werner, D-55130 Mainz (DE); SCHÖLKENS, Bernward, D-65779 Kelkheim (DE); FALK, Eugen, D-60529 Frankfurt (DE)
(86) Internationale Anmeldenummer: PCT/EP1997/002548
(87) Internationale Veröffentlichungsnummer: WO 1997/046226

(56) Entgegenhaltungen:
- EP-A- 0 612 723
- EP-A- 0 627 413
- EP-A- 0 640 593
- WO-A-96/04241
- US-A- 5 110 817
- US-A- 5 132 324
- SCHNEIDER J.: "Effects of a furosemide-amiloride combination on plasma lipoproteins in patients with mild to moderate hypertension and preexisting hyperlipoproteinemia" CURR. THER. RES. CLIN. EXP., 1990, 47/1 (239-244), USA, XP002040458
- TAMAGAKI ET AL.: "Effects of high density lipoproteins on intracellular pH and proliferation of human vascular endothelial cells" ATHEROSCLEROSIS, Bd. 123, Nr. 1-2, 1.Juni 1996, Seiten 73-82, XP002048401
- W. SCHOLZ ET AL.: "Effects of Na+/H+ exchange inhibitors in cardiac ischemia" MOLECULAR AND CELLULAR CARDIOLOGY, Bd. 24, Nr. 7, Juli 1992, Seiten 733-741, XP002048402
- J.-R. NOFER ET AL.: "Low-density lipoproteins inhibit the Na+/H+ antiport in human platelets" CIRCULATION, Bd. 95, Nr. 6, 18.März 1997, Seiten 1370-1377, XP002048403
- DATABASE PHARMAPROJECTS PJB Publications Dialog File 928 Accession N0. 24561, 12.Juli 1996 "Pharmaprojects No. 4929" XP002040459

## Beschreibung

Verwendung von Inhibitoren des zellulären Na⁺/H⁺-Exchangers (NHE) zur Herstellung eines Medikament zur Normalisierung der Serumlipide

Die Erfindung betrifft die Verwendung NHE-Inhibitoren zur Herstellung eines Medikaments zur Normalisierung der Serumlipide.

Bei den bekannten und als NHE-Inhibitoren identifizierten Wirkstoffen handelt es sich um Guanidin-Derivate, vorzugsweise um Acylguanidine, unter anderem wie sie in folgenden Publikationen und Patentveröffentlichungen beschrieben: Edward J. Cragoe, Jr., "DIURETICS, Chemistry, Pharmacology and Medicine", J. WILEY & Sons (1983), 303 - 341, außerdem um Verbindungen der folgenden Formeln: (HOE 89/F 288 - US 5 292 755)
a) Benzoylguanidine der Formel I 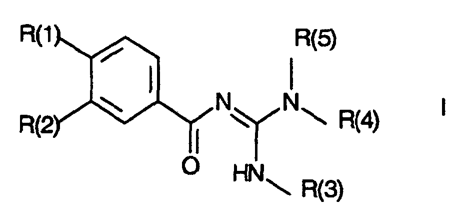 worin bedeuten:
   R(1) oder R(2)
   R(6)-S(O)ₙ- oder R(7)R(8)N-O₂S-;
   und der jeweils andere Substituent R(1) oder R(2)
   H, F, Cl, Br, (C₁-C₄)-Alkyl, (C₁-C₄)-Alkoxy oder Phenoxy,
   das unsubstituiert ist oder substituiert mit 1 - 3 Substituenten ausgewählt aus der Gruppe bestehend aus Fluor, Chlor, Methyl und Methoxy;
   oder der jeweils andere Substituent R(1) oder R(2)
   R(6)-S(O)ₙ oder R(7)R(8)N-;
   n Null, 1 oder 2;
   R(6) (C₁-C₆)-Alkyl, (C₅-C₇)-Cycloalkyl, Cyclopentylmethyl, Cyclohexylmethyl oder Phenyl,
   das unsubstituiert ist oder substituiert mit 1 - 3 Substituenten ausgewählt aus der Gruppe bestehend aus Fluor, Chlor, Methyl und Methoxy;
   R(7) und R(8)
   gleich oder verschieden H oder (C₁-C₆)-Alkyl;
   oder
   R(7) Phenyl-(CH₂)ₘ;
   m 1 - 4;
   oder
   R(7) Phenyl,
   das unsubstituiert ist oder substituiert mit 1 - 2 Substituenten ausgewählt aus der Gruppe bestehend aus Fluor, Chlor, Methyl und Methoxy;
   oder
   R(7) und R(8)
   gemeinsam eine geradkettige oder verzweigte (C₄-C₇)-Kette, wobei die Kette zusätzlich durch O, S oder NR(9) unterbrochen sein kann;
   R(9) H oder Methyl;
   oder
   R(7) und R(8)
   gemeinsam mit dem Stickstoffatom, an das sie gebunden sind, ein Dihydroindol-, Tetrahydrochinolin- oder Tetrahydroisochinolin-System;
   R(3), R(4) und R(5)
   unabhängig voneinander H oder (C₁-C₂)-Alkyl,
   oder
   R(3) und R(4)
   gemeinsam eine (C₂-C₄)-Alkylenkette;
   oder
   R(4) und R(5)
   gemeinsam eine (C₄-C₇)-Alkylenkette;
   sowie deren pharmazeutisch verträgliche Salze;
   (HOE 92/F 303 K - EP-OS589 336, NZ 248 703)
f) Benzoylguanidine der Formel I 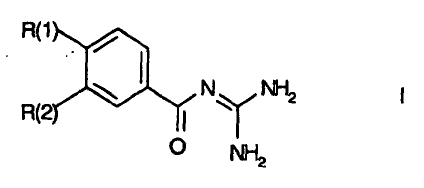 worin bedeuten:
   R(1) oder R(2)
   R(3)-S(O)ₙ- oder 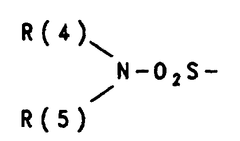 der jeweils andere Substituent R(1) oder R(2)
   H, OH, F, Cl, Br, J, C₁-C₄-Alkyl, C₁-C₄-Alkoxy, Benzyloxy oder Phenoxy,
   das unsubstituiert ist oder einen bis drei Substituenten ausgewählt aus der Gruppe bestehend aus Fluor, Chlor, Methyl, Methoxy, Hydroxy oder Benzyloxy trägt,
   R(3)-S(O)ₙ, -NR4)R(5) oder 3,4-Dehydropiperidin
   R(3) C₁-C₆-Alkyl, C₅-C₇-Cycloalkyl, Cyclopentylmethyl, Cyclohexylmethyl oder Phenyl,
   das unsubstituiert ist oder einen bis drei Substituenten ausgewählt aus der Gruppe bestehend aus Fluor, Chlor, Methyl und Methoxy trägt;
   R(4) und R(5)
   gleich oder verschieden, H oder C₁-C₆-Alkyl;
   oder
   R(4) Phenyl-(CH₂)ₘ-;
   m 1, 2, 3 oder 4;
   oder
   R(4) Phenyl,
   das unsubstituiert ist oder einen bis zwei Substituenten ausgewählt aus der Gruppe bestehend aus Fluor, Chlor, Methyl und Methoxy trägt;
   oder
   R(4) und R(5)
   gemeinsam eine geradkettige oder verzweigte C₄-C₇-Kette, wobei die Kette zusätzlich durch O, S oder NR(6) unterbrochen sein kann,
   R(6) H oder Methyl;
   oder
   R(4) und R(5)
   gemeinsam mit dem Stickstoffatom, an das sie gebunden sind, ein Dihydroindol, Tetrahydrochinolin oder Tetrahydroisochinolin-System;
   n Null, 1 oder 2;
sowie deren pharmazeutisch verträgliche Salze.

Für derartige Inhibitoren des Na⁺/H⁺-Austauschs werden bereits zahlreiche medizinische Verwendungen beschrieben, wie beispielsweise Krankheitsformen, die durch chronische oder akute Blutunterversorgung eines Organs (Ischämie), insbesondere des Herzens, entstehen. Sie sind deshalb geeignet beispielsweise zur Behandlung ischämisch induzierter Arrhythmien, unterschiedlicher Formen der Angina Pectoris, bei Herztransplantationen, in der Herzchirurgie und bei angioplastischen chirurgischen Eingriffen. Weitere beschriebene Indikationen für NHE-Inhibitoren sind Schlaganfall und Hirnödem, Schock und proliferationsbedingte Krankheiten, wie Atherosklerose, diabetische Spätschäden, fibrotische Erkrankungen und Organhypertrophien.

Es wurde nun überraschend gefunden, daß NHE-Inhibitoren eine günstige Beeinflussung der Serumlipoproteine zeigen. Es ist allgemein anerkannt, daß für die Entstehung arteriosklerotischer Gefäßveränderungen, insbesondere der koronaren Herzkrankheit, zu hohe Blutfettwerte, sogenannte Hyperlipoproteinämien, einen wesentlichen Risikofaktor darstellen. Für die Prophylaxe und die Regression von atherosklerotischen Veränderungen kommt daher der Senkung erhöhter Serum-Lipoproteine eine außerordentliche Bedeutung zu. Neben der Reduktion des Serum-Gesamtcholesterins kommt der Senkung des Anteils spezifischer atherogener Lipidfraktionen dieses Gesamtcholesterins, insbesondere der low density Lipoproteine (LDL) und der very low density Lipoproteine VLDL) eine besondere Bedeutung zu, da diese Lipidfraktionen einen atherogenen Risikofaktor darstellen. Dagegen wird den high density Lipoproteinen eine Schutzfunktion gegen die koronare Herzkrankheit zugeschrieben. Dementsprechend sollen Hypolipidämika in der Lage sein, nicht nur das Gesamtcholesterin, sondern insbesondere die VLDL und LDL-Serumcholesterinfraktionen zu senken. Es wurde nun überraschend gefunden, daß NHE-Inhibitoren bezüglich der Beeinflussung der Serumlipidspiegel wertvolle therapeutisch verwertbare Eigenschaften zeigen. So erniedrigen sie signifikant die erhöhten Serumkonzentration von LDL und VLDL, wie sie beispielweise durch erhöhte diätetisch Einnahme einer cholesterin- und lipidreichen Kost oder bei pathologischen Stoffwechselveränderungen, beispielsweise genetisch bedingten Hyperlipidämien zu beobachten sind. Sie können daher zur Prophylaxe und zur Regression von atherosklerotischen Veränderungen herangezogen werden, indem sie einen kausalen Risikofaktor ausschalten. Hierzu zählen nicht nur die primären Hyperlipidämien, sondern auch gewisse sekundäre Hyperlipidämien, wie sie z.B. beim Diabetes vorkommen. Darüber hinaus führen die NHE-Inhibitoren zu einer deutlichen Reduktion der durch Stoffwechselanomalien induzierten Infarkte und insbesondere zu einer signifikanten Verminderung der induzierten Infarktgröße und dessen Schweregrades. Weiterhin führen NHE-Inhibitoren zu einen wirksamen Schutz gegen Schädigungen durch Stoffwechselanomalien induzierter Endothelschädigungen. Mit diesem Schutz der Gefäße gegen das Syndrom der endothelialen Dysfunktion sind NHE-Inhibitoren wertvolle Arzneimittel zur Prävention und zur Behandlung koronarer Gefäßspasmen, der Atherogenese und der Atherosklerose, der linksventrikulären Hypertrophie und der dilatierten Kardiomyopathie, und thrombotischer Erkrankungen.

Diese antihyperlipidämische Wirkung wird für einen NHE-Inhibitor, das 4-Isopropyl-3-methylsulfonylbenzoyl-guanidin Methansulfonat (Hoe 642) in der nachfolgenden Tabelle gezeigt, wobei männliche New Zealand weiße Neuseeländerkaninchen (n=28) mit einem Gewicht von 3 - 4 kg verwendet und in vier nachfolgend beschriebene randomisierte Gruppen von jeweils 7 Tieren eingeteilt wurden: 1) Standard- Kaninchenfutter (®Altromin 2834), 2) atherogene Diät mit einem Gehalt von 0,25% Cholesterin und 3% Kokosnußöl, 3) Standard Kaninchenfutter + Hoe 642 (0,1%), 4) atherogene Diät + Hoe 642 (0,1%).

Die Entnahme der Blutproben erfolgte einmalig nach 30 tägiger Fütterung durch Punktion der Ohrarterie. Aus dem Blut wurde Serum gewonnen. Die Messung des Gesamtcholesterins erfolgte aus dem Serum mittels der CHOD (Cholesterinoxidase)-PAP Methode (Merckotest, Merck Diagnostika, E. Merck, 64271 Darmstadt, Germany). Die Trennung der Lipoproteine erfolgte, ebenfalls aus dem Serum durch FPLC (Fast Protein Liquid Chromatography)-Methode (März et al. 1993, Clin. Chem. 39/11:2276-2281).

### Ergebnisse

Gesamtcholesterinwerte sowie die Subfraktionen VLDL, LDL und HDL aus den Kaninchenseren:

| (mmol/l) | Cholesterin | VLDL | LDL | HDL |
|---|---|---|---|---|
| | | | | |
| Normaldiät | 0.65 ± 0.08 | 0.05 ± 0.01 | 0.12 ± 0.02 | 0.48 ± 0.06 |
| | | | | |
| Normaldiät +HOE642 | 0.69 ±0.1 | 0.05 ± 0.02 | 0.23 ± 0.04 | 0.41 ± 0.17 |
| | | | | |
| Cholesterindiät | 17.85 ± 3.71 | 5.68 ± 1.38 | 9.31 ± 1.88 | 2.86 ± 0.73 |
| | | | | |
| Cholesterindiät + HOE 642 | 7.95 ± 0.89* | 4.6 ± 0.6 | 2.1 ± 0.2* | 1.8 ± 0.2 |

Die Werte sind als Mittelwerte ± SEM (mittlerer Fehler des Mittelwertes) angegeben. Signifikanzen: *p<0.05 Cholesterindiät + HOE 642 vs Cholesterindiät. Zwischen den beiden Normaldiätgruppen gibt es keine signifikanten Unterschiede.
Beide Normaldiätgruppen sind zu den beiden Cholesteringruppen signifikant unterschieden.

Die erfindungsgemäß verwendeten Verbindungen finden deshalb vorteilhaft Verwendung zur Herstellung eines Medikaments zur Behandlung von Hypercholesterinämie; zur Herstellung eines Medikaments zur Prävention der Atherogenese; zur Herstellung eines Medikaments zur Prävention und Behandlung der Atherosklerose, zur Herstellung eines Medikaments zur Prävention und Behandlung von Krankheiten, die durch erhöhte Cholesterinspiegel ausgelöst werden, zur Herstellung eines Medikaments zur Prävention und Behandlung von Krankheiten; die durch endotheliale Dysfunktion ausgelöst werden, zur Herstellung eines Medikaments zur Prävention und Behandlung von atherosklerose-induzierter Hypertonie, zur Herstellung eines Medikaments zur Prävention und Behandlung von atherosklerose-induzierter Thrombosen, zur Herstellung eines Medikaments zur Prävention und Behandlung von Hypercholesterinämie- und endothelialer Dysfunktion induzierter ischämischer Schäden und postischämischer Reperfusionsschäden, zur Herstellung eines Medikaments zur Prävention und Behandlung von Hypercholesterinämie- und endothelialer Dysfunktion induzierter cardialer Hypertrophien und Cardiomyopathien, zur Herstellung eines Medikaments zur Prävention und Behandlung von Hypercholesterinämie- und endothelialer Dysfunktion induzierter koronarer Gefäßspasmen und myocardialer Infarkte, zur Herstellung eines Medikaments zur Behandlung der genannten Leiden in Kombinationen mit blutdrucksenkenden Stoffen, bevorzugt mit Angiotensin Converting Enzyme (ACE)-Hemmern und Angiotensin-Rezeptorantagonisten, eine Kombination eines NHE-Inhibitors mit einem blutfettspiegelsenkenden Wirkstoff, bevorzugt mit einem HMG-CoA-Reduktaseinhibitor, z. B. Lovastatin oder Pravastatin, wobei letzterer eine hypolipidämische Wirkung herbeiführt und dadurch die hypolipidämischen Eigenschaften des NHE-Inhibitors steigert, erweist sich als günstige Kombination mit verstärkter Wirkung und vermindertem Wirkstoffeinsatz.

Beansprucht wird die Gabe von Natrium-Protonen-Austausch-Hemmern als neuartige Arzneimittel zur Senkung erhöhter Blutfettspiegel, sowie die Kombination von Natrium-Protonen-Austausch-Hemmern mit blutdrucksenkenden und/oder hypolipidämisch wirkenden Arzneimittel.

## Patentansprüche

1. Verwendung eines Na⁺/H⁺-Exchange Inhibitors zur Herstellung eines Medikaments zur Behandlung von erhöhten Blutfettspiegeln, **dadurch gekennzeichnet, daß** man als Na⁺/H⁺-Exchange Inhibitor einsetzt:
Benzoylguanidine der Formel I 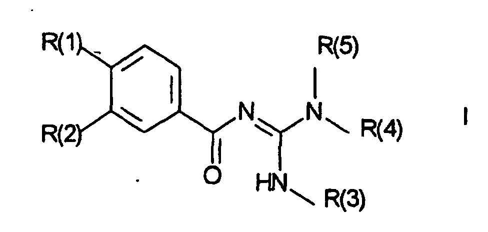 worin bedeuten:
R(1) oder R(2)
R(6)-S(O)ₙ- oder R(7)R(8)N-O₂S-;
und der jeweils andere Substituent R(1) oder R(2)
H, F, Cl, Br, (C₁-C₄)-Alkyl, (C₁-C₄)-Alkoxy oder Phenoxy,
das unsubstituiert ist oder substituiert mit 1 - 3 Substituenten ausgewählt aus der Gruppe bestehend aus Fluor, Chlor, Methyl und Methoxy;
oder der jeweils andere Substituent R(1) oder R(2)
R(6)-S(O)ₙ oder R(7)R(8)N-;
n Null, 1 oder 2;
R(6) (C₁-C₆)-Alkyl, (C₅-C₇)-Cycloalkyl, Cyclopentylmethyl, Cyclohexylmethyl oder Phenyl,
das unsubstituiert ist oder substituiert mit 1 - 3 Substituenten ausgewählt aus der Gruppe bestehend aus Fluor, Chlor, Methyl und Methoxy;
R(7) und R(8)
gleich oder verschieden H oder (C₁-C₆)-Alkyl;
oder
R(7) Phenyl-(CH₂)ₘ;
m 1 - 4;
oder
R(7) Phenyl,
das unsubstituiert ist oder substituiert mit 1 - 2 Substituenten ausgewählt aus der Gruppe bestehend aus Fluor, Chlor, Methyl und Methoxy;
oder
R(7) und R(8)
gemeinsam eine geradkettige oder verzweigte (C₄-C₇)-Kette, wobei die Kette zusätzlich durch O, S oder NR(9) unterbrochen sein kann;
R(9) H oder Methyl;
oder
R(7) und R(8)
gemeinsam mit dem Stickstoffatom, an das sie gebunden sind, ein Dihydroindol-, Tetrahydrochinolin- oder Tetrahydroisochinolin-System;
R(3), R(4) und R(5)
unabhängig voneinander H oder (C₁-C₂)-Alkyl,
oder
R(3) und R(4)
gemeinsam eine (C₂-C₄)-Alkylenkette;
oder
R(4) und R(5)
gemeinsam eine (C₄-C₇)-Alkylenkette;
sowie deren pharmazeutisch verträgliche Salze;
oder
Benzoylguanidine der Formel I 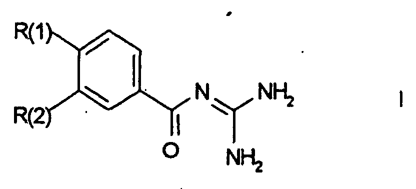 worin bedeuten:
R(1) oder R(2)
R(3)-S(O)ₙ- oder 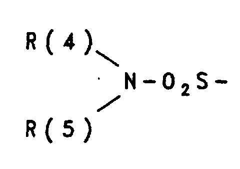 der jeweils andere Substituent R(1) oder R(2)
H, OH, F, Cl, Br, J, C₁-C₄-Alkyl, C₁-C₄-Alkoxy, Benzyloxy oder Phenoxy, das unsubstituiert ist oder einen bis drei Substituenten ausgewählt aus der Gruppe bestehend aus Fluor, Chlor, Methyl, Methoxy, Hydroxy oder Benzyloxy trägt,
R(3)-S(O)ₙ, -NR4)R(5) oder 3,4-Dehydropiperidin
R(3) C₁-C₆-Alkyl, C₅-C₇-Cycloalkyl, Cyclopentylmethyl, Cyclohexylmethyl oder Phenyl,
das unsubstituiert ist oder einen bis drei Substituenten ausgewählt aus der Gruppe bestehend aus Fluor, Chlor, Methyl und Methoxy trägt;
R(4) und R(5)
gleich oder verschieden, H oder C₁-C₆-Alkyl;
oder
R(4) Phenyl-(CH₂)ₘ-;
m 1, 2, 3 oder 4;
oder
R(4) Phenyl,
das unsubstituiert ist oder einen bis zwei Substituenten ausgewählt aus der Gruppe bestehend aus Fluor, Chlor, Methyl und Methoxy trägt;
oder
R(4) und R(5)
gemeinsam eine geradkettige oder verzweigte C₄-C₇-Kette, wobei die Kette zusätzlich durch O, S oder NR(6) unterbrochen sein kann,
R(6) H oder Methyl;
oder
R(4) und R(5)
gemeinsam mit dem Stickstoffatom, an das sie gebunden sind, ein Dihydroindol, Tetrahydrochinolin oder Tetrahydroisochinolin-System;
n Null, 1 oder 2;
sowie deren pharmazeutisch verträgliche Salze;

2. Verwendung eines Na⁺/H⁺-Exchange Inhibitors nach Anspruch 1 zur Herstellung eines Medikaments zur Prävention und Behandlung der Atherosklerose.

3. Verwendung eines Na⁺/H⁺-Exchange Inhibitors nach Anspruch 1 zur Herstellung eines Medikaments zur Behandlung von hypercholesterinämiebedingten Erkrankungen des Herz-Kreislaufsystems.

4. Verwendung eines Na⁺/H⁺-Exchange Inhibitors nach Anspruchen 1 zur Herstellung eines Medikaments zur Prävention und Behandlung des endothelialen Dysfunktionssyndroms.

5. Verwendung eines Na ⁺/H ⁺-Exchange Inhibitors nach Anspruch 1 zur Herstellung eines Medikaments zur Prävention und Behandlung cardialer Hypertrophien und Cardiomyopathien.

6. Verwendung eines Na ⁺/H ⁺-Exchange Inhibitors nach Anspruch 1 zur Herstellung eines Medikaments zur Prävention und Behandlung koronarer Gefäßspasmen und myocardialer Infarkte, die infolge Atherosklerose erhöhter Lipidspiegel und endotheliales Dysfunktionssyndrom verursacht werden.

7. Verwendung eines Na ⁺/H⁺-Exchange Inhibitors nach Anspruch 1 zur Herstellung eines Medikaments zur Prävention und Behandlung Thrombosen, die durch Atherosklerose erhöhter Lipidspiegel und endotheliales Dysfunktionssyndrom verursacht werden.

8. Verwendung eines Na⁺/H⁺-Exchange Inhibitors nach Anspruch 1 zur Herstellung eines Medikaments zur Prävention und Behandlung von Bluthochdruck, der durch Atherosklerose, erhöhte Lipidspiegel und endotheliales Dysfunktionssyndrom verursacht wird.

9. Verwendung eines Na⁺/H⁺-Exchange Inhibitors nach Anspruch 1 zur Herstellung eines Medikaments zur Prävention und Behandlung peripherer Gefäßkrankheiten, die durch Atherosklerose, erhöhte Lipidspiegel und endotheliales Dysfunktionssyndrom verursacht werden.

10. Verwendung eines Na ⁺/H⁺-Exchange Inhibitors zur Herstellung eines Medikaments nach Ansprüchen 1 bis 9 in Kombinationen mit einem blutdrucksenkenden Medikament.

11. Verwendung eines Na ⁺/H⁺-Exchange Inhibitors zur Herstellung eines Medikaments nach Ansprüchen 1 bis 9 in Kombinationen mit einem Angiotensin Converting Enzyme (ACE)-Hemmer.

12. Verwendung eines Na ⁺/H ⁺-Exchange Inhibitors zur Herstellung eines Medikaments nach Ansprüchen 1 bis 9 in Kombinationen mit einem Angiotensin Rezeptorantagonisten.

13. Verwendung eines Na⁺/H⁺-Exchange Inhibitors zur Herstellung eines Medikaments nach Ansprüchen 1 bis 9 in Kombinationen mit einem blutfettspiegelsenkenden Wirkstoff.

14. Verwendung eines Na⁺/H⁺-Exchange Inhibitors zur Herstellung eines Medikaments nach Ansprüchen 1 bis 9 in Kombinationen mit einem HMG-CoA-Reduktaseinhibitor.

15. Verwendung eines Na⁺/H⁺-Exchange Inhibitors zur Herstellung eines Medikaments nach Ansprüchen 1 bis 9 in Kombinationen mit Lovastatin oder Pravastatin.

## Claims

1. Use of a Na⁺/H⁺ exchange inhibitor for the production of a medicament for the treatment of raised blood lipid levels, which comprises employing as Na⁺/H⁺ exchange inhibitor:
benzoylguanidines of the formula I 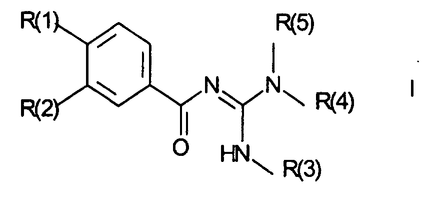 in which:
R(1) or R(2)
is R(6)-S(O)ₙ- or R(7)R(8)N-O₂S-;
and the other substituent R(1) or R(2) in each case
is H, F, Cl, Br, (C₁-C₄)-alkyl, (C₁-C₄)-alkoxy or phenoxy,
which is unsubstituted or substituted by 1 - 3 substituents selected from the group consisting of fluorine, chorine, methyl and methoxy;
or the other substituent R(1) or R(2) in each case
is R(6)-S(O)ₙ or R(7)R(8)N-;
n is zero, 1 or 2;
R(6) is (C₁-C₆)-alkyl, (C₅-C₇)-cycloalkyl, cyclopentylmethyl, cyclohexylmethyl or phenyl,
which is unsubstituted or substituted by 1 - 3 substituents selected from the group consisting of fluorine, chlorine, methyl and methoxy;
R(7) and R(8)
identically or differently are H or (C₁-C₆)-alkyl;
or
R(7) is phenyl-(CH₂)ₘ;
m is 1 - 4;
or
R(7) is phenyl,
which is unsubstituted or substituted by 1 - 2 substituents selected from the group consisting of fluorine, chlorine, methyl and methoxy;
or
R(7) and R(8)
together are a straight-chain or branched (C₄-C₇)-chain, where the chain can additionally be interrupted by O, S or NR(9);
R(9) is H or methyl;
or
R(7) and R(8)
together with the nitrogen atom to which they are bonded, are a dihydroindole, tetrahydroquinoline or tetrahydroisoquinoline system;
R(3), R(4) and R(5)
independently of one another are H or (C₁-C₂)-alkyl,
or
R(3) and R(4)
together are a (C₂-C₄)-alkylene chain;
or
R(4) and R(5)
together are a (C₄-C₇)-alkylene chain;
and their pharmaceutically tolerable salts;
or
benzoylguanidines of the formula I 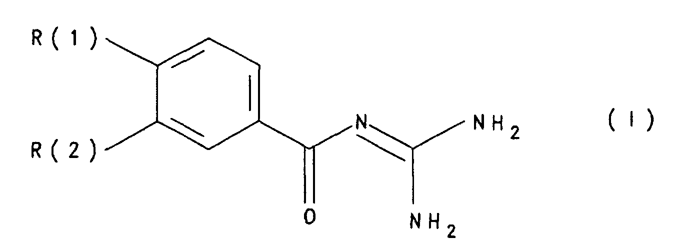 in which:
R(1) or R(2)
is R(3)-S(O)ₙ- or 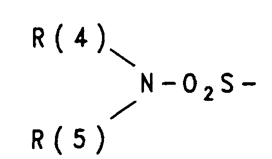 the other substituent R(1) or R(2) in each case
is H, OH, F, Cl, Br, I, C₁-C₄-alkyl, C₁-C₄-alkoxy, benzyloxy or phenoxy,
which is unsubstituted or carries one to three substituents selected from the group consisting of fluorine, chlorine, methyl, methoxy, hydroxyl and benzyloxy,
R(3)-S(O)ₙ, -NR(4)R(5) or 3,4-dehydropiperidine
R(3) is C₁-C₆-alkyl, C₅-C₇-cycloalkyl, cyclopentylmethyl, cyclohexylmethyl or phenyl,
which is unsubstituted or substituted by one to three substituents selected from the group consisting of fluorine, chlorine, methyl and methoxy;
R(4) and R(5)
identically or differently, are H or C₁-C₆-alkyl;
or
R(4) is phenyl-(CH₂)ₘ-;
m is 1, 2, 3 or 4;
or
R(4) is phenyl,
which is unsubstituted or carries one to two substituents selected from the group consisting of fluorine, chlorine, methyl and methoxy;
or
R(4) and R(5)
together are a straight-chain or branched C₄-C₇-chain, where the chain can additionally be interrupted by O, S or NR(6),
R(6) is H or methyl;
or
R(4) and R(5)
together with the nitrogen atom to which they are bonded, are a dihydroindole, tetrahydroquinoline or tetrahydroisoquinoline system;
n is zero, 1 or 2;
and their pharmaceutically tolerable salts;

2. The use of a Na⁺/H⁺ exchange inhibitor as claimed in claim 1 for the production of a medicament for the prevention and treatment of atherosclerosis.

3. The use of a Na⁺/H⁺ exchange inhibitor as claimed in claim 1 for the production of a medicament for the treatment of hypercholesterolemia-related disorders of the cardiovascular system.

4. The use of a Na⁺/H⁺ exchange inhibitor as claimed in claim 1 for the production of a medicament for the prevention and treatment of endothelial dysfunction syndrome.

5. The use of a Na⁺/H⁺ exchange inhibitor as claimed in claim 1 for the production of a medicament for the prevention and treatment of cardiac hypertrophies and cardiomyopathies.

6. The use of a Na⁺/H⁺ exchange inhibitor as claimed in claim 1 for the production of a medicament for the prevention and treatment of coronary vascular spasms and myocardial infarcts, which are caused as a result of atherosclerosis, increased lipid levels and endothelial dysfunction syndrome.

7. The use of a Na⁺/H⁺ exchange inhibitor as claimed in claim 1 for the production of a medicament for the prevention and treatment of thromboses which are caused by atherosclerosis as a result of increased lipid levels and endothelial dysfunction syndrome.

8. The use of a Na⁺/H⁺ exchange inhibitor as claimed in claim 1 for the production of a medicament for the prevention and treatment of high blood pressure which is caused by atherosclerosis, increased lipid levels and endothelial dysfunction syndrome.

9. The use of a Na⁺/H⁺ exchange inhibitor as claimed in claim 1 for the production of a medicament for the prevention and treatment of peripheral vascular disorders which are caused by atherosclerosis, increased lipid levels and endothelial dysfunction syndrome.

10. The use of a Na⁺/H⁺ exchange inhibitor for the production of a medicament as claimed in claims 1 to 9 in combination with a hypotensive medicament.

11. The use of a Na⁺/H⁺ exchange inhibitor for the production of a medicament as claimed in claims 1 to 9 in combination with an angiotensin converting enzyme (ACE) inhibitor.

12. The use of a Na⁺/H⁺ exchange inhibitor for the production of a medicament as claimed in claims 1 to 9 in combination with an angiotensin receptor antagonist.

13. The use of a Na⁺/H⁺ exchange inhibitor for the production of a medicament as claimed in claims 1 to 9 in combination with a blood lipid level-lowering active compound.

14. The use of a Na⁺/H⁺ exchange inhibitor for the production of a medicament as claimed in claims 1 to 9 in combination with an HMG-CoA reductase inhibitor.

15. The use of a Na⁺/H⁺ exchange inhibitor for the production of a medicament as claimed in claims 1 to 9 in combination with lovastatin or pravastatin.

## Revendications

1. Utilisation d'un inhibiteur de l'échange Na⁺/H⁺ en vue de la préparation d'un médicament pour le traitement de niveaux élévés de lipides sanguins, **caractérisée en ce qu'**on utilise comme inhibiteur de l'échange Na⁺/H⁺ :
des benzoylguanidines de formule I 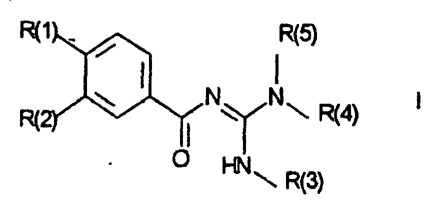 dans laquelle :
R(1) ou R(2)
signifie R(6)-S(O)ₙ- ou R(7)R(8)N-O₂S- ;
et l'autre substituant R(1) ou R(2)
signifie à chaque fois H, F, Cl, Br, alkyle en C₁ à C₄, alcoxy en C₁ à C₄ ou phénoxy,
non substitué ou substitué par 1 à 3 substituants choisis dans le groupe constitué par fluor, chlore, méthyle et méthoxy ;
ou l'autre substituant R(1) ou R(2)
signifie à chaque fois R(6)-S(O)ₙ ou R(7)R(8)N- ;
n vaut zéro, 1 ou 2 ;
R(6) signifie alkyle en C₁ à C₆, cycloalkyle en C₅ à C₇, cyclopentylméthyle, cyclohexylméthyle ou phényle,
non substitué ou substitué par 1 à 3 substituants choisis dans le groupe constitué par fluor, chlore, méthyle et méthoxy ;
R(7) et R(8)
sont identiques ou différents et signifient H ou alkyle en C₁ à C₆ ;
ou
R(7) signifie phényl-(CH₂)ₘ ;
m vaut 1 à 4 ;
ou
R(7) signifie phényle,
non substitué ou substitué par 1 à 2 substituants choisis dans le groupe constitué par fluor, chlore, méthyle et méthoxy ;
ou
R(7) et R(8)
signifient ensemble une chaîne en C₄ à C₇ linéaire ou ramifiée, la chaîne pouvant en outre être interrompue par O, S ou NR(9) ;
R(9) signifie H ou méthyle ;
ou
R(7) et R(8)
signifient ensemble avec l'atome d'azote auquel ils sont liés un système dihydroindole, tétrahydroquinoléine ou tétrahydroisoquinoléine ;
R(3), R(4) et R(5)
signifient, indépendamment l'un de l'autre, H ou alkyle en C₁ à C₂ ;
ou
R(3) et R(4)
signifient ensemble une chaîne alkyle en C₂ à C₄ ;
ou
R(4) et R(5)
signifient ensemble une chaîne alkyle en C₄ à C₇ ;
ainsi que leurs sels pharmaceutiquement acceptables ;
ou
des benzoylguanidines de formule I 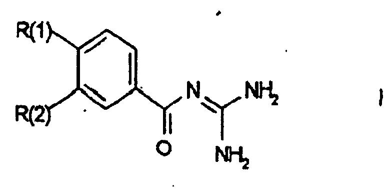 dans laquelle :
R(1) ou R(2)
signifie R(3)-S(O)ₙ- ou 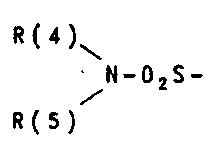 l'autre substituant R(1) ou R(2)
signifie à chaque fois H, OH, F, Cl, Br, I, alkyle en C₁ à C₄, alcoxy en C₁ à C₄, benzyloxy ou phénoxy,
non substitué ou portant un à trois substituants choisis dans le groupe constitué par fluor, chlore, méthyle, méthoxy, hydroxy ou benzyloxy,
R(3)-S(O)ₙ, -NR(4)R(5) ou 3,4-déshydropipéridine
R(3) signifie alkyle en C₁ à C₆, cycloalkyle en C₅ à C₇, cyclopentylméthyle, cyclohexylméthyle ou phényle,
non substitué ou portant un à trois substituants choisis dans le groupe constitué par fluor, chlore, méthyle et méthoxy ;
R(4) et R(5)
sont identiques ou différents et signifient H ou alkyle en C₁ à C₆ ;
ou
R(4) signifie phényl-(CH₂)ₘ- ;
m vaut 1, 2, 3 ou 4 ;
ou
R(4) signifie phényle,
non substitué ou portant un à deux substituants choisis dans le groupe constitué par fluor, chlore, méthyle et méthoxy ;
ou
R(4) et R(5)
signifient ensemble une chaîne en C₄ à C₇ linéaire
ou ramifiée, la chaîne pouvant en outre être interrompue par O, S ou NR(6)
R(6) signifie H ou méthyle ;
ou
R(4) et R(5)
signifient ensemble avec l'atome d'azote auquel ils sont liés un système dihydroindole, tétrahydroquinoléine ou tétrahydroisoquinoléine ;
n vaut zéro, 1 ou 2 ;
ainsi que leurs sels pharmaceutiquement acceptables.

2. Utilisation d'un inhibiteur de l'échange Na⁺/H⁺ selon la revendication 1 pour la préparation d'un médicament destiné à la prévention et au traitement de l'athérosclérose.

3. Utilisation d'un inhibiteur de l'échange Na⁺/H⁺ selon la revendication 1 pour la préparation d'un médicament destiné au traitement des maladies du système cardiovasculaire provoquées par l'hypercholestérolémie.

4. Utilisation d'un inhibiteur de l'échange Na⁺/H⁺ selon la revendication 1 pour la préparation d'un médicament destiné à la prévention et au traitement du syndrome de dysfonctionnement endothélial.

5. Utilisation d'un inhibiteur de l'échange Na⁺/H⁺ selon la revendication 1 pour la préparation d'un médicament destiné à la prévention et au traitement d'hypertrophies cardiaques et de cardiomyopathies.

6. Utilisation d'un inhibiteur de l'échange Na⁺/H⁺ selon la revendication 1 pour la préparation d'un médicament destiné à la prévention et au traitement des spasmes vasculaires coronaires et des infarctus du myocarde, qui sont provoqués par l'athérosclérose, un niveau de lipides élevé et le syndrome de dysfonctionnement endothélial.

7. Utilisation d'un inhibiteur de l'échange Na⁺/H⁺ selon la revendication 1 pour la préparation d'un médicament destiné à la prévention et au traitement de thromboses, qui sont provoquées par l'athérosclérose, un niveau de lipides élevé et le syndrome de dysfonctionnement endothélial.

8. Utilisation d'un inhibiteur de l'échange Na⁺/H⁺ selon la revendication 1 pour la préparation d'un médicament destiné à la prévention et au traitement de la hypertension artérielle, qui est provoquée par l'athérosclérose, un niveau de lipides élevé et le syndrome de dysfonctionnement endothélial.

9. Utilisation d'un inhibiteur de l'échange Na⁺/H⁺ selon la revendication 1 pour la préparation d'un médicament destiné à la prévention et au traitement de maladies des vaisseaux périphériques, qui sont provoquées par l'athérosclérose, un niveau de lipides élevé et le syndrome de dysfonctionnement endothélial.

10. Utilisation d'un inhibiteur de l'échange Na⁺/H⁺ pour la préparation d'un médicament selon les revendications 1 à 9 en association avec un médicament abaissant la tension sanguine.

11. Utilisation d'un inhibiteur de l'échange Na⁺/H⁺ pour la préparation d'un médicament selon les revendications 1 à 9 en association avec un inhibiteur de l'enzyme de conversion de l'angiotensine (ACE).

12. Utilisation d'un inhibiteur de l'échange Na⁺/H⁺ pour la préparation d'un médicament selon les revendications 1 à 9 en association avec des antagonistes des récepteurs de l'angiotensine.

13. Utilisation d'un inhibiteur de l'échange Na⁺/H⁺ pour la préparation d'un médicament selon les revendications 1 à 9 en association avec une substance active diminuant le niveau de lipides sanguins.

14. Utilisation d'un inhibiteur de l'échange Na⁺/H⁺ pour la préparation d'un médicament selon les revendications 1 à 9 en association avec un inhibiteur de la HMG-CoA-réductase.

15. Utilisation d'un inhibiteur de l'échange Na⁺/H⁺ pour la préparation d'un médicament selon les revendications 1 à 9 en association avec la lovastatine ou la pravastatine.
